# EUROPEAN PATENT SPECIFICATION

(11) **EP 1 834 635 B1**
(45) Date of publication and mention of the grant of the patent: **06.07.2011**
(21) Application number: 06380048.6
(22) Date of filing: 13.03.2006
(51) Int. Cl.: A61K 9/51, A61K 8/11, B01J 13/02

(54) **Stable nanocapsule systems for the administration of active molecules**
Stabile Nanokapselsysteme für die Verabreichung von Wirkstoffen
Systèmes de nanocapsules stables pour l'administration de principes actifs

(43) Date of publication of application: 19.09.2007
(73) Proprietor: Advanced in Vitro Cell Technologies, S.L., 08028 Barcelona (ES)
(72) Inventor: Vila Pena, Ana Isabel Pabellon de Servicios, 15782 Santiago de Compostella (ES); Suárez Luque, Silvia Pabellon de Servicios, 15782 Santiago de Compostella (ES); Alonso Fernández, M. Jose Pabellon de Servicios, 15782 Santiago de Compostella (ES)
(74) Representative: ABG Patentes, S.L.

(56) References cited:
- EP-A- 0 771 566
- EP-A- 1 243 323
- PREGO ET AL: "Chitosan-PEG nanocapsules as new carriers for oral peptide delivery" JOURNAL OF CONTROLLED RELEASE, ELSEVIER, AMSTERDAM, NL, vol. 111, no. 3, 14 February 2006 (2006-02-14), pages 299-308, XP005351968 ISSN: 0168-3659
- CALVO PILAR ET AL: "Evaluation of cationic polymer-coated nanocapsules as ocular drug carriers" 1997, INTERNATIONAL JOURNAL OF PHARMACEUTICS (AMSTERDAM), VOL. 153, NR. 1, PAGE(S) 41-50 , XP002394165 ISSN: 0378-5173 * page 42, paragraph 2.1 * * page 43, paragraph 2.2 * * page 45; table 1 *

## Description

### FIELD OF THE INVENTION

The invention is aimed at nanocapsulate systems characterized in that they have high stability during long periods of time. These systems are useful for the controlled release of pharmacologically or cosmetically active molecules, especially of highly lipophilic nature. It is specifically aimed at systems which comprise nanocapsules dispersed in an aqueous phase, cosmetic and pharmaceutical compositions which comprise them, as well as processes for their preparation.

### STATE OF THE ART

Systems for the release of biologically active agents constitute a field of research in continuous development. It is widely known in the field of galenic medicine that the administration of active ingredients, especially those of highly lipophilic nature, by topical route (transdermal or via the mucous) is particularly unsuitable, due to the fact that their high lipophilicity means that penetration through the epidermal lipid barrier is practically impossible.

For this reason, in the last two decades, different systems have been developed for the release of this type of molecules which permit increasing the bioavailability and absorption of these drugs via the epithelium after their application. From among these systems principally stands out the preparation of colloidal suspensions such as liposomes, microemulsions, nanospheres and nanoparticles, based on the formation of an oil phase wherein the active ingredient is incorporated, dispersed in an aqueous phase thanks to the use of one or several surfactant agents.

Thus, Prego et al. [J. Control. Release, 111 (2006) 299-308] disclose an aqueous colloidal suspension of nanocapsules with a lipophilic core made of lecithin and an oil, which comprises C₈-C₁₀ chain fatty acids (e.g. Miglyol 812), and a hydrophilic coating comprising chitosan-PEG and Poloxamer 188.
Application EP1243323 discloses nanocapsules with a lipophilic matrix core comprising phospholipids, such as lecithin, and emulsifiers, such as C₁₂-C₂₄ chain fatty acid esters or polymer derivatives thereof, and a hydrophilic coating of chitosan.Calvo et al. [J. Pharm. Pharmacol., 1996, 48, 1147-52] disclose colloidal systems for the release of indomethacin by ocular route, consisting of an oil phase of lecithin and poly-ε-caprolactone dispersed in an aqueous phase which contains poloxamer as a surfactant agent.

For its part, application EP 760 237 relates to microemulsions for the release of water-insoluble substances, such as cyclosporine, which contains a lipophilic dispersed phase, consisting of triglycerides, lecithin and a surfactant, and an aqueous hydrophilic phase which contains propylene glycol.

Application WO97/22358 discloses microemulsions wherein cyclosporine is dissolved in a system which comprises a hydrophobic phase, consisting of tocopherol or derivatives thereof, and a series of hydrophilic components such as propylene carbonate and polyethylene glycol, as well as a surfactant.

Nevertheless, the main drawback of this type of colloidal systems is that they have a negative net charge, so that their contact with certain biological components of cationic character (some protein, sodium and potassium ions) causes the neutralization of the surface charge, the rupture of the system and, consequently, the loss of the active ingredient. On the other hand, these negatively charged systems may also undergo an electrostatic repulsion by the biological membranes, due to them also having a negative charge.

In order to overcome this limitation, important research work has been performed for the purpose of developing colloidal systems which have a positive net charge and which, therefore, allow them to be absorbed via biological environments of cationic character and/or their adhesion to biological membranes (mucous and skin).

Application WO96/37232 relates to systems based on nanoemulsions, nanocapsules and nanoparticles consisting of a lipophilic nucleus and coated with chitosan for the administration of active ingredients by different routes. The incorporation of the hydrophilic chitosan polymer in the aqueous phase confers the positive net charge to said system. Since then, systems have been developed which incorporate this polymer or others which allow the desired positive charge to be provided.

Thus, Calvo et al. [Colloid. Polym. Sci., 1997, 275, 46-53] disclose the same colloidal systems of the application mentioned in the previous paragraph but further incorporating poloxamer as surfactant agent in the aqueous phase, to release lipophilic molecules using diazepam as model. In the same line, the scientific publication of El-Shabouri [Intern. J. Pharmac., 2002, 249, 101-8] is developed, wherein, in addition to chitosan, the use of sodium glycolate or gelatine-A in the aqueous phase, and its capacity for cyclosporine release, is analysed.

Likewise, the article by Filipovic-Grcic [J. Microencap., 2001, 18(1), 3-12] relates to mucoadhesive systems based on a liposomal nucleus coated with chitosan wherein a high molecular weight marker is incorporated (fluorescein isothiocyanate-dextran), analysing the stability of said system in a simulated gastric fluid, in accordance with the proportion of chitosan and its molecular weight.

Nevertheless, an important technical problem associated to the use of this type of systems in the field of medicine and cosmetology, is that related to its instability, both during its storage and after its *in vivo* administration. Specifically, the composition of these systems causes, during storage for time periods longer than a month, and at temperatures above room temperature, the active ingredient to be gradually released from the lipophilic phase and move to the aqueous phase wherein it is insoluble, leading to its precipitation and the formation of crystals.

In light of this data, the development of stable nanocapsule systems is especially necessary, particularly for the release of active lipophilic ingredients and which have, in addition to a great capacity for the association of these molecules and their release in the appropriate biological environment, a stability which is clearly improved during its storage and transportation, essential characteristics from the point of view of industrial applicability.

Another technical problem is that most of these systems are unstable when they undergo sterilization processes, such as autoclaving or pasteurization. These processes involve subjecting the systems to temperatures over 100°C for short periods of time. In general, these strong physical conditions drastically change the solubility of the different components and lead to the rupture of emulsions or particles, with the formation of an oil phase where the active ingredient would be found. It would therefore be highly desirable to find a system for the controlled release of active ingredients or cosmetic ingredients with lipophilic character, which can be administered topically and which is stable even when subjected to sterilization processes.

### BRIEF DESCRIPTION OF THE INVENTION

The inventors have found that a system of nanocapsules which comprise a lipophilic nucleus which comprises a phospholipid component and an oil consisting of saturated and/or unsaturated C₁₂-C₂₄ chain fatty acids or derivatives thereof, and by a hydrophilic phase which comprises chitosan and a polyoxyalkylenated compound, has high stability in suspension for periods of time up to more than two years in normal storage conditions, which represents a key aspect of the industrial and commercial feasibility of these systems. It further permits an efficient association and subsequent release of highly lipophilic molecules.

An additional advantage derived from these systems is their capacity to be subjected to a sterilization process at high temperatures maintaining their structure and properties, such as particle size and charge measured by zeta potential. This aspect is fundamental when its use is required, for example, in ocular applications where sterilization is necessary. Furthermore, the possibility of sterilizing these systems permits the reduction, and in some cases, the elimination of the need to use preservatives in the final formulations.

Thus, an object of the present invention consists of a system which comprises nanocapsules with an average size less than 1 µm dispersed in an aqueous medium, wherein the nanocapsules have a structure which comprises
a) a lipophilic phase which comprises:
   a.1) at least one phospholipid component; and
   a.2) an oil which comprises one or more saturated and/or unsaturated C₁₂-C₂₄ chain fatty acids or derivatives thereof;
b) a hydrophilic phase which comprises:
   b.1) chitosan or a derivative thereof; and
   b.2) a polyoxyalkylenated compound.

A second object of the invention relates to a pharmaceutical composition which comprises a system such as that previously defined and which further comprises an active molecule capable of preventing, relieving or curing diseases.

Another object of the invention relates to a cosmetic composition which comprises a system such as that previously defined. In a variant of the invention, said cosmetic composition can further incorporate a cosmetically active molecule.

A final object of the invention relates to a process for the preparation of a system such as that previously defined which comprises:
a) preparation of the lipophilic phase by the dissolution of the phospholipid component and the oil in an organic solvent, and optionally a lipophilic molecule as active ingredient;
b) preparation of the hydrophilic phase by the dissolution in water of the chitosan and the polyoxyalkylenated compound;
c) heating of both phases to a temperature between 40 and 60°C;
d) mixing the lipophilic and hydrophilic phase with spontaneous formation of the nanocapsules;
e) elimination of the organic solvent.

The systems and compositions of the invention are especially suitable for the administration of biologically active molecules with a lipophilic character, and which are incorporated in the lipophilic phase of the nanocapsules.

### DETAILED DESCRIPTION OF THE INVENTION

The system of the present invention comprises nanocapsules which are dispersed in an aqueous medium, where said nanocapsules have a structure which comprises a lipophilic phase, wherein an active ingredient can be incorporated, and a hydrophilic phase.

The term "nanocapsule" is understood as a nucleus of lipophilic nature surrounded by a hydrophilic phase which comprises chitosan with positive charge which "encapsulates" the nucleus. The nanocapsule is dispersed in an aqueous medium, where said aqueous medium may comprise dissolved additional quantities of chitosan and/or of other hydrophilic polymers. The ionic interaction resulting between the different lipophilic and hydrophilic components of the nanocapsule generates characteristic physical entities, which are independent and observable, whose average size is less than 1 µm, i.e. an average size between 1 and 999 nm.

"Average size" is understood as the average diameter of the nanocapsule population, which comprise the lipophilic phase and the hydrophilic phase, which moves together in the aqueous medium. The average size of these systems can be measured using standard procedures known by a person skilled in the art, and which are described, for example, in the experimental part below.

The nanocapsules of the system of the invention are characterized in that they have an average particle size of less than 1 µm, preferably they have an average size between 1 and 999 nm, preferably between 50 and 800 nm. The average size of the capsules is mainly influenced by the quantity of the phospholipid component (in larger quantities, the resulting size is smaller), by the quantity and molecular weight of the chitosan (the size increases with a greater quantity or molecular weight), the density of the oil used, and by parameters of the preparation process, such as stirring rate and temperature of both phases in the mixing process.

Furthermore, the nanocapsules may have a surface charge (measured by zeta potential) attributed to the amine groups of the chitosan, whose magnitude may vary between +1 mV and +70 mV.

### Lipophilic phase

The lipophilic phase which is present in the nanocapsule comprises at least one phospholipid component and an oil consisting of one or several saturated and/or unsaturated long chain fatty acids (C₁₂-C₂₄), or derivatives thereof. Its function is that of housing the active lipopholic ingredient when it is present, both if it is active in the pharmacological and cosmetic sense, which will later be released in the suitable biological environment.

### Phospholipid component

In the present description phospholipid component is understood to be a type of ionic lipid, which in its most simple form are comprised of a glycerol, whereto two fatty acids (1,2-diacylglycerol) and a phosphate group are bonded. Usually, the phosphate group is bonded by a phosphodiester bond to another group of atoms, which frequently contain nitrogen, such as choline, serine or ethanolamine and many times has an electric charge. Other phospolipids are derived from the aminoalcohol sphingosine or dihydrosphingosine (sphingolipids), and not necessarily from glycerol. The resulting compound has a lipophilic part for the chains of the fatty acids and a hydrophilic part for the phosphate group and the electric charge.

In the system of the invention the phospholipid component is preferably selected from phosphoglycerides, sphingolipids, cephalin and mixtures thereof. The phosphoglycerides, such as phosphatidylcholine, phosphatidylethanolamine, phosphatidylserine, phosphatidylglycerine, phosphatidylinositol, lysophosphatidylcholine and phosphatidic acid, are lipids formed by fatty acids, glycerine, phosphoric acid and alcohol (e.g. choline, serine, ethanolamine, glycerine or inositol). Sphingolipids such as sphingomyelins are formed by sphingosine, a fatty acid, phosphoric acid and an alcohol or aminoalcohol. Cephalin is formed from glycerine, phosphoric acid, two fatty acids and two alcohol bases (choline and sphingosine).

In a preferred embodiment of the invention, the phospholipid component comprises phosphoglycerides. More preferably it comprises phosphoglycerides selected from phosphatidylcholine, phosphatidylethanolamine, lysophosphatidylcholine and phosphatidic acid or mixtures thereof.

In a preferred variant, the phospholipid component is lecithin. Preferably commercially available soy lecithin is used, although egg lecithin can also be used.

Lecithin is a complex mixture of lipid compounds, wherein the majority are phospholipid compounds, specifically phosphatidylcholine, phosphatidylethanolamine and lysophosphatidylcholine. In general, lecithins are designated by their commercial name and a number which indicated the percentage of phosphatidylcholine in their composition. The percentage of the phosphatidylcholine in the lecithins may vary between 35% up to practically 100%, nevertheless in the present invention lecithins are preferably used wherein this percentage is between 35% and 75%, preferably between 35% and 55%. The use of lecithins with a very high percentage of phosphatidylcholine (over 80%) is not advisable as it gives rise to the presence of free oil in the nanocapsule suspension. In this regard, there are several lecithins available on the market which have the characteristic of a phosphatidylcholine percentage of no more than 80%. Thus, preferably Epikuron 135F, Epikuron 145V, Epikuron 170, Lipoid S30, Lipoid S45 and Lipoid S75 are used.

Likewise, the phosphatidylcholine/phosphatidylethanolamine ratio influences the formation of nanocapsules with suitable stability properties. Thus, it has been demonstrated that the use of Epikuron 135F, wherein the phosphatidylcholine/phosphatidylethanolamine ratio is approximately 7/1 and the proportion of phosphatidylcholine is greater than 35% in the composition, provides the best stability results.

### Oil

For its part, the oil comprised in the lipophilic phase consists of one or several unsaturated and/or saturated C₁₂-C₂₄ chain fatty acids or derivatives thereof. Examples of said fatty acids include, although it is not limited to them, saturated fatty acids such as lauric acid, myristic acid, palmitic acid, stearic acid, acacidic acid, carnacilic acid, araquidic acid, behenic acid, lignoceric acid or cerotic acid; monounsaturated fatty acids such as lauroleic acid, palmitoleic acid, oleic acid, vaccenic acid, gadoleic acid, cetoleic acid, or erucic acid; polyunsaturated fatty acids such as linoleic acid, linolenic acid, gamma linolenic acid, stearidonic acid, araquidonic acid, clupanodonic acid or docosahexaenoic acid.

Likewise, derivates of these fatty acids can be used, understanding by this definition those compounds with a highly lipophilic character and produced as a consequence of the reaction of the acid group with alcohols or amines, such as, for example, the esters or amides of said fatty acids. In the same way, the definition of fatty acid derivates include those fatty acids, their esters or their amides which have hydroxyl, ether groups or alkyl groups as substituents of the hydrocarbon chain, such as, for example, cerebronic acid, hydroxynervonic acid, ricinoleic acid, tuberculostearic acid or phytanic acid; or those fatty acids which have a cyclic structure in the hydrocarbon chain, such as lactobacillic acid or chaulmogric acid.

In general terms, any vegetable oil can be used, such as, for example, sunflower oil, corn oil, soy oil, cotton oil, peanut oil, soy oil, evening primrose oil, borage oil, olive oil, coconut oil, palm oil, hempseed oil, apricot kernel or seed oil, wheatgerm oil, corn germ oil, cocoa butter oil, macadamia oil, almond oil, avocado oil, calendula oil, grapeseed oil, sesame oil, jojoba oil, hazelnut oil, etc.

Surprisingly, it has been found that the incorporation of this type of oil in the nanocapsules' composition is particularly relevant to give the system high stability during long periods of time if it is compared with other oils used in the state of the art, such as the oils commercially called Migliol or Labrafac. In particular, it has been observed using assays simulated in accelerated conditions that the system of the invention is stable during 5-6 months, at temperatures around 37°C, which is equivalent to almost two years in normal storage conditions at room temperature. When this is compared with the results of the other type of oils such as Migliol 840 and Labrafac Lipophile (consisting of a mixture of diesters or triglycerides of saturated C₈-C₁₀ chain fatty acids) it has been seen that system stability increases up to 6 times.

In a preferred embodiment of the invention, the oil comprised in the lipophilic phase is castor oil. Said oil is obtained by cold-pressing *Ricinus Communis L.* seeds, however, it is a commercially available product. It has a composition of long-chain fatty acids (C₁₆-C₁₈): 2% (maximum) of palmitic acid, 2.5% (maximum) of stearic acid, 2.5-6.0% of oleic acid, 2.5-7.0% of linoleic acid 1% (maximum) of linolenic acid and 85-92% of ricinoleic acid.

### Hydrophilic phase

The hydrophilic phase comprises chitosan or a derivative thereof and a polyoxyalkylenated compound which acts as surfactant agent, in addition to water. The function of this phase is probably that of coating the nanocapsule, in addition to providing it with a positive charge which allows it to be absorbed through biological environments of cationic character or absorption on them.

### Chitosan

Chitosan is a polymer of natural origin derived from chitin (poly-N-acetyl-D-glucosamine), where an important part of the acetyl groups of the N have been eliminated by hydrolysis. The degree of deacetylation is preferably greater than 40%, more preferably greater than 60%. In a variant it is between 60-98%. It therefore has an aminopolysaccharide structure and cationic character. It comprises the repetition of monomeric units of formula (I): 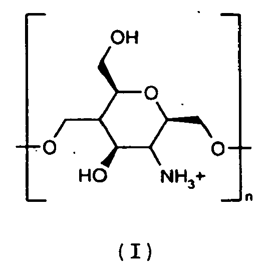 wherein n is an integer, and furthermore m units where the amine group is acetylated. The sum of n+m represents the degree of polymerization, i.e. the number of monomeric units in the chitosan chain.

The chitosan used to produce the nanocapsules of the present invention has a molecular weight between 2 and 2000 kDa, preferably between 2 and 500 kDa, more preferably between 5 and 150 kDa. Examples of commercial chitosans which may be used are UPG 113, UP CL 213 and UP CL113 which may be obtained from NovaMatrix, Drammen, Norway.

As an alternative to chitosan, a derivative thereof can also be used, understanding as such a chitosan wherein one or more hydroxyl groups and/or one or more amine groups have been modified, with the aim of increasing the solubility of the chitosan or increasing the adhesive nature thereof. These derivatives include, among others, acetylated, alkylated or sulfonated chitosans, thiolated derivatives, as is described in Roberts, Chitin Chemistry, Macmillan, 1992, 166. Preferably, when a derivative is used it is selected from 0-alkyl ethers, O-acyl esters, trimethyl chitosan, chitosans modified with polyethylene glycol, etc. Other possible derivatives are salts, such as citrate, nitrate, lactate, phosphate, glutamate, etc. In any case, a person skilled in the art knows how to identify the modifications which can be made on the chitosan without affecting the stability and commercial feasibility of the end formulation.

### Polyoxyalkylenated compound

For its part, polyoxyalkylenated compound is understood to mean a synthetic hydrophilic polymer of nonionic character which has units of alkylene oxide in its structure, being preferred the use of a polyoxyethylene (PEO) or an ethylene oxide-propylene oxide (PEO-PPO) copolymer. In this last case, when a copolymer of PEO and PPO is used, the proportion of PEO in the copolymer may range from between 10% and 80% by weight, the rest (up to 100%) being PPO.

These polymers are marketed with different molecular weights, nevertheless, in the present invention the use of polyoxyethylenated derivatives which have molecular weights between 1000 and 25000 is preferred. In a preferred embodiment, the polyoxyethylenated derivative is a triblock copolymer (PEO-PPO-PEO), such as, for example, those commercially called Poloxamer. Good results have been obtained for example with Pluronic F68 (Poloxamer 188).

The polyoxyalkylenated compound is used as surfactant agent in the system and its presence is particularly necessary to confer stability thereto, as otherwise, the nanocapsules aggregate during the storage. Thus, in its absence, an increase in particle size has been observed after two weeks at 37°C. Furthermore, it has been possible to demonstrate that the incorporation of this compound hardly affects the size of the nanocapsules formed as can be observed in table I.

**Table I: Influence of the quantity of poloxamer on nanocapsule stability**

| poloxamer (mg) | Initial size | size after 2 weeks at 37°C |
|---|---|---|
| 0 | 492 | 754 |
| 125 | 482 | 475 |
| 205 | 439 | 442 |
| 500 | 410 | 423 |

In a variant of the invention, the system has a proportion of phospholipid component which is preferably between 0.1% and 10% by weight with respect to the total weight of the system including the water, preferably between 0.5% and 5%, more preferably between 1% and 2.5% by weight. Proportions less than 0.1% of the phospholipid component would not be recommendable since it would lead to the presence of free oil in the nanocapsule suspension; in contrast, a proportion above 10% could lead to the formation of particles and not encapsulated systems due to the chitosan interaction with positive charge-lipid component with negative charge.

On the other hand, in another variant of the invention the proportion of oil in the system ranges from 1%-20%, preferably from 2.5% and 10%, by weight with respect to the total weight of the system including the water. Smaller quantities involve the reduction of active ingredient which can be encapsulated, whilst larger quantities make it necessary to increase the proportion of other components, such as the phospholipid component, to avoid the presence of free oil in the system.

In another variant of the invention the proportion of chitosan, or of a derivative thereof, is preferably between 0.05% and 10% by weight with respect to the total weight of the system including the water, preferably between 0.1% and 2%, and more preferably between 0.25% and 1%. Proportions larger than those mentioned cause an increase in viscosity of the suspension, which may be advantageous for some applications but considerably hinders the solvent elimination phase and formula concentration.

For its part, the proportion of polyoxyalkylenated compound is preferably between 0.05% and 10% by weight, preferably between 0.1% and 5% by weight, more preferably between 1% and 2.5%.

In all the cases, the remaining proportion largely corresponds to purified water where the nanocapsules are dispersed. This does not mean that other ingredients may not be present, either in the nanocapsules (one or more lipophilic active ingredients, cosmetic ingredients, etc), in the aqueous medium where they are dispersed (viscosing agents, preservatives, fragrances, etc) or absorbed on them (hydrophilic active ingredients, hyaluronic acid, etc).

In a variant of the system of the invention, the nanocapsules are dispersed in an aqueous medium which further comprises a hydrophilic polymer, whose function is to give viscosing properties to the system, increasing to a great extent the density thereof. The addition of this component is due to the nanocapsule system dispersed in aqueous medium of the invention resulting in some cases too fluid from the point of view of its application, especially when one wants to manufacture semi-solid pharmaceutical or cosmetic compositions such as gels, creams, ointments or balms. Among the hydrophilic polymers with viscosing character the following can be used, among others, carbohydrates and derivatives, carboxylic acids, poly glycols such as polyvinyl alcohol, derivatives of polyvinylpyrrolidone, chitosan or a derivative thereof, poloxamers, polyethylene glycol, hyaluronic acid or mixtures thereof.

Another variant of the invention incorporates, after the formation of the nanocapsules, hyaluronic acid or its salts in the aqueous medium, due to its different properties and functions. On the one hand, it is absorbed on the nanocapsules varying some of its properties such as the charge. On the other, it has been seen that it can encourage interaction with cell membranes and mucin (protein present in the mucous). Additionally, depending on the quantity used, it can remain dissolved in the aqueous medium and contributes to modulating the system's viscosity. In any case, it has been seen that it does not negatively affect the system's properties, in particular the measurements of size and zeta potential of the system, as can be seen in table II.

**Table II. Size and zeta potential of the nanocapsules after the incorporation of hyaluronic acid.**

| chitosan: hyaluronic acid (mg) | size (nm) | Zeta potential (mV) |
|---|---|---|
| 50:0 | 330 | +61.6 |
| 50:10 | 312 | +51.0 |
| 50:20 | 372 | +38.1 |
| 50:30 | 481 | +29.3 |

One of the more interesting applications of the system of the invention is for the administration of lipophilic molecules, especially when they are pharmacologically or cosmetically active compounds. The systems described in the invention have high capacity for the association of biologically active lipophilic molecules inside the nanocapsules.

Therefore, another object of the present invention consists of a pharmaceutical composition which comprises the previously defined nanocapsule system and a lipophilic molecule capable of preventing, relieving or curing diseases.

Examples of pharmaceutical compositions include any liquid composition (i.e. suspension or dispersion of nanocapsules) for its oral, buccal, sublingual, topical, ocular, nasal or vaginal application, or any composition in the form of gel, ointment, cream or balm for its topical, ocular, nasal or vaginal administration.

In a preferred aspect, the formulation is administered via the mucous. The positive surface charge the nanocapsules have provides a better absorption of the drugs on the mucous surface via their interaction with the mucous and the surfaces of the epithelial cells which are negatively charged.

In another preferred aspect, the formulation is administered intradermally or transdermally, understanding as such, administration through or on the skin. This form of administration is particularly relevant since the system of the invention not only encourages the absorption and penetration of the drugs by transdermal route, but also the adhesion of the system on the dermal surface and subsequent controlled release of the active component ("depot" type).

The term "biologically active lipophilic molecule" relates to any substance of lipophilic character which is used in the treatment, cure, prevention or diagnosis of a disease or which is used to improve the physical and mental well-being of humans and animals. These molecules with lipophilic character may include proteins, peptides, lipids, modified oligonucleotides (lipophilized), corticosteroids, lipophilic vitamins, such as A, D, K and E, antifungal agents, bacteriostatic agents, healing agents, antihistaminic agents, anaesthetic agents, antipruritic agents, antipsoriatic agents, antibiotic agents, antiviral agents, antiseptic agents, antiacne agents, depigmenting agents, antiseborrheic agents, immunosuppressant agents, non-steroidal anti-inflammatory drugs (NSAIDs), Capsicum-derived anti-inflammatory drugs.

In a preferred embodiment, the biologically active lipophilic molecule is cyclosporine.

In another preferred embodiment the biologically active lipophilic molecule is a corticosteroid such as, for example, hydrocortisone, prednisone, fluticasone, prednisolone, triamcinolone, triamcinolone acetonide, dexamethasone, betamethasone, beclomethasone, beclomethasone dipropionate.

In a preferred variant, the corticosteroid is betamethasone valerate.

Due to its good properties for administration on or through the skin, and due to its long-term stability characteristics, the systems of the invention are also very suitable for cosmetic applications.

Therefore an additional object of the present invention consists of a cosmetic composition which comprises the previously defined nanocapsule system. These cosmetic compositions include any liquid composition (suspension or dispersion of nanocapsules) or any composition which comprises the system of the invention and which is in the form of gel, cream, ointment or balm for its topical administration. Said compositions are characterized in that they have emollient, protective and healing properties even when they do not have any cosmetically active molecule associated.

In a variant of the invention, the cosmetic composition may also incorporate active molecules of lipophilic nature which, although they do not have any therapeutic effect, they have properties as a cosmetic agent. Among the active molecules which may be incorporated in the nanocapsules one can cite emollient agents, preservatives, fragrance substances, antiacne agents, antifungal agents, antioxidants, deodorants, antiperspirants, antidandruff agents, depigmenters, antiseborrheic agents, dyes, suntan lotions, UV light absorbers, enzymes, fragrance substances, among others.

The proportion of active ingredient encapsulated in the nanocapsules may come to be up to 5% by weight with respect to the total weight of the system including the water; larger quantities would reduce the stability of the system and would make it necessary to add a greater quantity of phospholipid component to encapsulate all the active ingredient. Nevertheless, the suitable proportion will depend in each case on the active ingredient to be encapsulated. In the specific case of encapsulating cyclosporine as active ingredient, the proportion thereof in said system would be between 0.01% and 5% by weight, preferably between 0.1% and 2%. Nevertheless, when betamethasone valerate is incorporated, the percentage of this ingredient would be included in the system between 0.01% and 3%, preferably between 0.05% and 0.5%.

The pharmaceutical and cosmetic compositions may further comprise pH controlling agents, such as, for example, buffer agents, which avoid the pH of the composition reducing to values below 5, antioxidant agents, which inhibit the oxidation of the phospholipid component and the oil, as well as preservatives which avoid important structural changes in the formulation. Thus, in a variant, citrate buffer is used in the lipophilic phase, which has a dual function which permits controlling the pH and acting at the same time as antioxidant agent, and parabens in the hydrophilic phase as preservative agents.

Depending on their function, these additional components may be present in the phases which compose the nanocapsules or in the aqueous medium where the latter are dispersed or totally or partially absorbed on them. A person skilled in the art can determine which additional components can be used and if they are necessary, many of them being in common use in pharmaceutical and cosmetic compositions.

In another aspect of the invention the system which comprises the nanocapsules can also be used for the administration of active ingredients which do not have a highly lipophilic nature. In that case, they are encapsulated in the nanocapsules but are absorbed on the surface of the latter. This variant may be useful for the preparation of stable systems for the administration of biologically active molecules of hydrophilic character, such as peptides, proteins, hormones (insulin, LRH, LD, etc.), antigens/allergens (titanic toxoid, diphteria toxin, etc.) according to the objective is to generate an immune response or tolerance, antibodies, heparin (low molecular weight and non-fractionated), DNA, RNA and oligonucleotides, among others.

A final object of the invention is related to a process for the preparation of the system of the invention as defined above, and which comprises nanocapsules. Said process comprises, on the one hand, the preparation of the lipophilic phase by the dissolution of the phospholipid component and the oil in an organic solvent. The incorporation of the optional lipophilic active ingredient, either pharmaceutical or cosmetic, is performed by dissolution thereof in this lipophilic phase together with the phospholipid component and the oil, irrespective of the order in which the components are added in the solution.

The choice of the organic solvent will depend to a large extent on the active ingredient incorporated. Although the use of pharmaceutically acceptable organic solvents, such as, for example, ethanol and isopropanol is preferable, in some cases, such as when one wants to encapsulate cyclosporine, one resorts to the use of acetone, since solvents such as ethanol or isopropanol, with high affinity for cyclosporine, cause the diffusion of the active ingredient to the aqueous phase during the subsequent mixing of the aqueous phase and the lipophilic phase, as can be seen in the following table.

**Table III. Influence of the quantity of cyclosporine and type of organic solvent on nanocapsule formation**

| Cyclosporine load | organic solvent | temperature | chitosan | result |
|---|---|---|---|---|
| | ethanol | | | aggregates |
| 1% | isopropanol | | | aggregates |
| | acetone | 50°C | 100 mg | nanocapsules |
| 0.5% | acetone | | | nanocapsules |
| 2% | acetone | | | aggregates |

It is preferable to use the smallest possible quantity of organic solvent, since it will further have to be totally or partially eliminated during the rest of the process.

In a particular embodiment, the organic solvent is added to a quantity of phospholipid component between 0.005% and 0.015% by weight, a quantity of oil of approximately 0.025 % by weight and a quantity of active ingredient between 0.0025% and 0.005% by weight, all the percentages being measured with respect to the total weight of organic solvent.

On the other hand, the hydrophilic phase is prepared by dissolving the chitosan and polyoxyalkylenated compound in water. In a preferred embodiment, between 0.0005% and 0.002% by weight of chitosan and between 0.001% and 0.005% by weight of polyoxyalkylenated compound are dissolved in water, all the percentages being measured with respect to the total weight of water.

Next, both phases are heated to a temperature between 40° and 60°C, preferably to 50°C and then they are mixed. This is surprising since it is not to be expected that the nanocapsules, and a stable system, are formed in that range of temperatures. It has been possible to observe that if it is heated above that maximum, the resulting preparations would not be suitable due to the existence of free oil in the suspension. By contrast, if it is heated under 40°C the start of solidification of the lipophilic components is observed. This mixing stage can be performed both by adding the aqueous phase to the organic phase, and the organic phase to the aqueous phase, since the addition order does not affect the system characteristics. On mixing both phases, the nanocaposules are spontaneously formed by a solvent displacing process, wherein when one phase is incorporated in the other, the organic solvent diffuses to the aqueous phase, forming lipid droplets consisting of the phospholipid component, the oil and optionally the active ingredient. The latter are characterized in that they are highly negative, causing the immediate location of chitosan around them, by ionic interaction due to this polymer's positive charge, which also further causes the surface charge of the system to also be positive.

Once the nanocapsules have been formed, the organic solvent used is eliminated by any method known by a person skilled in the art. The elimination of the organic solvent is due to the fact that its presence is not advisable in pharmaceutical formulations.

Next, one of the processes typical in the preparation of nanocapsule systems is the concentration thereof to increase component concentration. In the present invention it has been observed that the initial volume can unexpectedly be concentrated to a volume 17 times less than that of nanocapsule formulation, below which the system could begin to lose stability.

After the elimination of the organic solvent or after the concentration stage, additional agents described above can be added (viscosing agents, hyaluronic acid or its salts, preservatives, etc).

If the system is going to be used for the administration of non-lipophilic biologically active molecules, they can be added after the formation of the nanocapsules or after the elimination of the organic solvent.

Furthermore, it has surprisingly been observed that the nanocapsule system dispersed in aqueous phase of the present invention may be subjected to a high temperature sterilization process without affecting its stability. Said process includes, for example, an autoclaving process, tyndallization, HTST (high temperature short time) and UHT (ultra high temperature). These processes are known by persons skilled in the art and are performed with the aim of being able to use the system of the invention in applications which need to be sterilized as is the case of application by ocular route. Likewise, another advantage derived from this characteristic is that of allowing the use of preservatives in the manufacturing of commercial formulas aimed at other routes of administration to be considerably reduced.

The conditions used in general for high-temperature sterilization may cause the melting of the nanocapsules and, therefore, the release of the active ingredient. Nevertheless, it has been possible to observe that the nanocapsules of the present invention maintain their size and surface charge after the sterilization process and, furthermore, the appearance of crystals of the active ingredient in the system is not observed. This reveals that there is no release of said active ingredient from the nanocapsules to the aqueous phase.

As an additional characteristic, it is extremely important that the systems which have been subjected to the sterilization process are also stable during long periods of time, their stability having been proven in accelerated tests for at least 4 months at 37°C, equivalent a more that one year in normal conditions, as is shown in the examples attached to the invention, thus confirming the industrial and commercial feasibility of these systems.

Below, some illustrative examples are described which reveal the characteristics and advantages of the invention.

### Examples

As process common to the examples detailed below, the nanocapsules have been characterized from the point of view of size, zeta potential (or surface charge), encapsulation efficacy and presence of cyclosporine A crystals. The nanocapsule stability studies were performed in a Heidolph stove (Titramax 1000) and the autoclaving processes in a Presoclave 75 (Selecta).

*Size Distribution* has been performed using photon correlation spectroscopy (PCS; Zeta Sizer, Nano series, Nano-ZS, Malvern Instruments, UK) obtaining average size values of the nanoparticle population.

*The Zeta potential* has been measured using Laser Doppler Anemometry (LDA; Zeta Sizer, Nano series, Nano-ZS, Malvern Instruments, UK). To determine the electrospheric mobility, the samples were diluted in Milli-Q water.

The *release of Cyclosporine A and Betamethasone valerate* from the chitosan nanocapsules has been visually evaluated, observing if there exists the formation of crystals in the aqueous phase and quantifying the cyclosporine concentration by HPLC (maximum solubility in external phase 16 µg/ml) or betamethasone valerate (maximum solubility in external phase 5 µg/ml), according to the active ingredient encapsulated. In this way, if the quantity quantified in the external phase is equal to the maximum solubility of active ingredient it is deduced that crystal formation exists, although they are not visible.

The chitosan (Protasan UP Cl 113) used in the examples is from NovaMatrix-FMC Biopolymer, the Cyclosporine A from LC Laboratories, the Betamethasone valerate from Guinama, the castor oil from Galindo, the phosphatidylcholine from Degussa (Epikuron 135F), the poloxamer 188 from BASF Corporation, the acetone from Vorquimica (Analema) and the remaining products used come from Sigma Aldrich, sucrose, glucose and mannitol.

### Example 1

### Stability study of chitosan nanocapsules with cyclosporine A in suspension at 37°C

Chitosan nanocapsules with cyclosporine A are prepared as described below:
a) Preparation of the oil phase with 250 mg of lecithin, 0.5 ml of castor oil, 50 or 100 mg of cyclosporine A and 25 ml of acetone.
b) Preparation of the aqueous phase with 50 mg of chitosan, 125 mg of poloxamer 188 and 100 ml of distilled water.
c) Both phases are heated to 50°C, and then the organic phase is added to the aqueous phase, the nanocapsules spontaneously forming.

Then, the acetone is eliminated by applying heat (37°C) in a vacuum and the remaining volume is then concentrated until 10 mL of water remains.

The nanocapsules resulting from that preparation have a nanometric size less than one micron, and more specifically between 200-700 nm, positive zeta potential (+10mV- +70mV), a cyclosporine A encapsulation efficacy of practically 100% (this was directly quantified after the degradation and extraction of the active compound, and indirectly after the quantification in the external aqueous phase).

Those nanocapsules were subjected to a stability study during which they were stored at 37°C for 6 months, measuring the particle size, the zeta potential and the presence of cyclosporine in the aqueous phase at 1-month intervals. Due to the fact that the cyclosporine is insoluble in water, in the case of instability the tendency is to precipitate and give rise to the appearance of visible crystals.

**Table I:**

| Characteristics of chitosan nanocapsules with cyclosporine A (1%) stored at 37°C (Mean ± SD). | | | |
|---|---|---|---|
| **Time at 37°C (months)** | **Size (nm)** | **Zeta potential (mV)** | **Presence of cyclosporine A crystals** |
| 0 | 474 ± 42 | +42 ± 3 | NO |
| 2 | 541 ± 26 | +37 ± 2 | NO |

As shown in Table I, the nanocapsules with 1% cyclosporine, maintain a diameter less than one micron, a positive zeta potential, and they are stable for at least 2 months at 37°C.

**Table II:**

| Characteristics of chitosan nanocapsules with cyclosporine A (0.5%) stored at 37°C (Mean ± ST). | | | | |
|---|---|---|---|---|
| **Time at 37°C (months)** | **size (nm)** | **Zeta potential (mV)** | **Presence of cyclosporine A crystals** | **Concentration in external aqueous phase** |
| 0 | 452 ± 11 | +36 ± 4 | NO | < 16 µg/ml |
| 2 | 601 ± 28 | +21 ± 5 | NO | < 16 µg/ml |
| 3 | 585 ± 13 | +30 ± 1 | NO | < 16 µg/ml |
| 4 | 490 ± 4 | +28 ± 2 | NO | < 16 µg/ml |
| 5 | 396 ± 4 | +29 ± 5 | NO | < 16 µg/ml |

As can be observed in Table II, the chitosan nanocapsules incorporating cyclosporine A (0.5%), are stable for at least 5 months at 37°C, maintaining a diameter under 1 µm, positive zeta potential and the absence of cyclosporine A crystal formation, indicating that the molecule remains in the oil nucleus of the nanocapsules. The system is stable with regard to size and potential for a minimum of 6 months (maximum time of the study).

### Example 2

### Stability study of chitosan nanocapsules with cyclosporine A to the autoclave process

Chitosan nanocapsules with Cyclosporine A were prepared according to the process described in example 1, and, then, an aliquot was subjected to an autoclave process (120°C for 20 min). The main objective of this study was to evaluate if the size and potential of the nanocapsules had been modified during the autoclave process.

**Table III:**

| Characteristics of chitosan nanocapsules with cyclosporine A after an autoclave process (Mean ± SD) | | | | |
|---|---|---|---|---|
| | **size (nm)** | **Zeta potential Z (mV)** | **Presence of cyclosporine A crystals** | **Concentration in external aqueous phase** |
| **Before the autoclave process** | 575 ± 5 | + 42.1 ± 0.7 | NO | < 16 µg/ml |
| **After the autoclave process** | 591 ± 11 | + 46.2 ± 0.8 | NO | < 16 µg/ml |

As can be observed in Table III, after the autoclave process, the nanocapsules maintain their size and surface charge. Furthermore, after the autoclave process, no cyclosporine A crystals appear, which reveals that there is no significant release of the active molecule from the nanocapsules to the aqueous phase.

### Example 3

### Stability study of chitosan nanocapsules with cyclosporine A autoclaved in suspension at 37°C

Chitosan nanocapsules with Cyclosporine A were prepared according to the process described in example 1, then they were autoclaved following the process described in example 2, and they were stored at 37°C for 5 months. With the objective of evaluating the stability of this system, particle size, zeta potential and the presence of cyclosporine crystals in the aqueous phase were measured at 1-month intervals.

**Table IV:**

| Characteristics of chitosan nanocapsules with cyclosporine A autoclaved and stored at 37°C (Mean ± SD). | | | | |
|---|---|---|---|---|
| **Time at 37°C (months)** | **size (nm)** | **Zeta potential (mV)** | **Presence of cyclosporine A crystals** | **Concentration in external aqueous phase** |
| 0 | 591 ± 11 | + 46 ± 1 | NO | < 16 µg/ml |
| 2 | 635 ± 15 | + 41 ± 1 | NO | < 16 µg/ml |
| 3 | 506 ± 17 | + 51 ± 1 | NO | < 16 µg/ml |
| 4 | 485 ± 9 | + 47 ± 1 | NO | < 16 µg/ml |

The nanocapsules with cyclosporine A, coated with chitosan, and autoclaved, are stable at 37°C for, at least, 4 months (Table IV).

### Example 4

### Preparation of chitosan nanocapsules with Betamethasone valerate

Chitosan nanocapsules were prepared with Betamethasone valerate as described below:
a) preparation of the oil phase with 250 mg of lecithin, 0.5 ml of castor oil, 10 mg of Betamethasone valerate and 25 ml of acetone.
b) preparation of the aqueous phase with 50 mg of chitosan, 125 mg of poloxamer 188 and 100 ml of distilled water.
c) both phases are heated to 50°C, and then the organic phase is added to the aqueous phase, spontaneously forming the capsules.

The emulsion obtained is concentrated (eliminating the organic solvent) to a final volume of 10 ml, applying heat (37°C) and vacuum.

The nanocapsules resulting from that preparation have a nanometric size lower than one micron, and more specifically between 200-600 nm), positive zeta potential (+25mV- +60mV), a betamethasone valerate encapsulation efficacy of 99.9% (this was indirectly quantified after quantification of the external aqueous phase). More specifically, the physicochemical characteristics and encapsulation efficacy of the nanocapsules are described in Table VI.

**Table VI:**

| Physicochemical characteristics and encapsulation efficacy of chitosan nanocapsules with Betamethasone valerate (Mean ± SD). | | |
|---|---|---|
| **Size (nm)** | **Zeta potential (mV)** | **Encapsulation Efficacy (%)** |
| 348 ± 71 | + 46.9 ± 1.3 | 99.94 ± 0.01 |

### Example 5

### Stability study of chitosan nanocapsules with Betamethasone valerate in suspension at room temperature

The nanocapsules prepared as indicated in example 4, were subjected to a stability study, wherein they were stored at room temperature for 6 months, measuring the particle size, zeta potential and the presence of betamethasone valerate in the aqueous phase. As shown in Table VII, they maintain nanometric size, positive zeta potential, and the betamethasone valerate remains in the lipid nuclei of the nanocapsules.

**Table VII:**

| Characteristics of chitosan nanocapsules with Betamethasone valerate stored at room temperature (Mean ± SD). | | | |
|---|---|---|---|
| **Time at room temp. (months)** | **size (nm)** | **Zeta potential (mV)** | **Concentration in external aqueous phase** |
| 0 | 383 ± 41 | + 46.9 ± 1.3 | < 5 µg/ml (0.6 µg/ml) |
| 6 | 413 ± 10 | + 48.2 ± 2,6 | < 5 µg/ml (0.9 µg/ml) |

### Example 6

### Stability study of chitosan nanocapsules with Betamethasone valerate in suspension at 37°C

The nanocapsules prepared as indicated in example 4, were subjected to a stability study, wherein they were stored at 37°C for 6 months, measuring the particle size, zeta potential and the presence of betamethasone valerate in the aqueous phase. Chitosan nanocapsules with Betamethasone valerate are stable

**Table VIII:**

| Characteristics of chitosan nanocapsules with Betamethasone valerate stored at 37°C (Mean ± SD). | | | |
|---|---|---|---|
| **Time at 37°C (months)** | **size (nm)** | **Zeta potential (mV)** | **Concentration in external aqueous phase** |
| 0 | 383 ± 41 | + 46.9 ± 1.3 | < 5 µg/ml (0.6 µg/ml) |
| 6 | 465 ± 14 | + 58.4 ± 1.8 | < 5 µg/ml (1.5 µg/ml) |

## Claims

1. A system which comprises nanocapsules with an average size less than 1 µm dispersed in an aqueous medium, wherein the nanocapsules have a structure which comprises:
a. a.lipophilic phase which comprises:
a.1 at least, a phospholipid component; and
a.2) an oil which comprises one or more saturated and/or unsaturated C₁₂-C₂₄ chain fatty acids or derivatives thereof;
b. a hydrophilic phase which comprises:
b.1 chitosan or a derivative thereof; and
b.2) a polyoxyalkylenated compound.

2. System according to claim 1, wherein the phospholipid component comprises phosphoglycerides, preferably selected from phosphatidylcholine, phosphatidylethanolamine, lysophosphatidylcholine, phosphatidic acid and mixtures thereof.

3. System according to claim 2, wherein the phospholipid component is lecithin, preferably soy lecithin.

4. System according to claim 3, wherein the lecithin has a phosphatidylcholine proportion between 35% and 75% by weight, preferably between 35% and 55%.

5. System according to any of claims 1 to 4., wherein the oil is castor oil.

6. System according to any of claims 1 to 5, wherein the polyoxyalkylenated compound is a polyoxyethylene or an ethylene oxide-propylene oxide copolymer.

7. System according to claim 6, wherein the polyoxyethylenated compound is a poloxamer, preferably it is poloxamer 188.

8. System according to any of claims 1 to 7, wherein the proportion of phospholipid component in the system is between 0.1% and 10% by weight with respect to the total weight including the water, preferably between 0.5% and 5%, more preferably between 1% and 2.5% by weight.

9. System according to any of claims 1 to 8, wherein the proportion of oil in the system is between 1% and 20% by weight with respect to the total weight including the water, preferably between 2.5% and 10%, more preferably between 4.5% and 5.5% by weight.

10. System according to any of claims 1 to 9, wherein the proportion of chitosan in the system is between 0.05% and 10% by weight with respect to the total weight including the water, preferably between 0.1% and 2%, more preferably between 0.25% and 1% by weight.

11. System according to any of claims 1 to 10, wherein the proportion of the polyoxyalkylenated compound in the system is between 0.05% and 10% by weight with respect to the total weight including the water, preferably between 0.1% and 5%, more preferably between 1% and 2.5%.

12. System according to any of claims 1 to 11, wherein the aqueous medium further comprises a viscosing hydrophilic polymer.

13. System according to claim 12, wherein the viscosing polymer is selected from between polyvinyl alcohol, polyvinylpyrrolidone, chitosan, poloxamers, polyethylene glycol and mixtures thereof.

14. System according to any of claims 1 to 13, which further comprises hyaluronic acid or any of its salts.

15. System according to any of claims 1 to 14, wherein the nanocapsules have an average size of between 1 and 999 nm, preferably between 50 and 800 nm, more preferably between 200 and.700 nm.

16. System according to any of claims 1 to 15, wherein the nanocapsules have a positive surface charge, preferably represented by a zeta potential between +1mV and +70 mV.

17. A system according to any of claims 1 to 16, which further comprises a lipophilic molecule in the lipophilic phase of the nanocapsules selected from the group consisting of proteins, peptides, lipids, lipophilized oligonucleotides, corticosteroids, lipophilic vitamins, such as A, D, K and E, antifungal agents, bacteriostatic agents, healing agents, antihistaminic agents, anaesthetic agents, antipruritic agents, antipsoriatic agents, antibiotic agents, antiviral agents, antiseptic agents, antiacne agents, depigmenting agents, antiseborrheic agents, immunosuppressant agents, non-steroidal antiinflammatory drugs (NSAIDs), Capsicum-derived anti-inflammatories.

18. System according to claim 17, wherein the lipophilic molecule is cyclosporine.

19. System according to claim 17, wherein the lipophilic molecule is hydrocortisone, prednisone, fluticasone, prednisolone, triamcinolone, triamcinolone acetonide, dexamethasone, betamethasone, betamethasone valerate, beclomethasone, beclomethasone dipropionate, preferably betamethasone valerate.

20. Pharmaceutical composition which comprises a system such as that defined in any of claims 1 to 19 and a biologically active molecule capable of preventing, relieving or curing diseases.

21. Composition according to claim 20, for administration by oral, buccal, sublingual, topical, ocular, nasal or vaginal route.

22. Cosmetic composition which comprises a system such as that defined in any of claims 1 to 19.

23. Composition according to claim 22, which comprises a cosmetically acceptable lipophilic molecule which is selected from emollient agents, preservatives, fragrance substances, antiacne agent, antifungal agents, antioxidants, deodorant, antiperspirants, antidandruff agents, depigmenters, antiseborrheic agents, dyes, suntan lotions, UV light absorbers, enzymes and fragrance substances.

24. Process for the preparation of a system according to any of claims 1 to 19, which comprises:
a. preparation of the lipophilic phase by dissolving the phospholipid component and the oil in the an organic solvent, and optionally a lipophilic molecule as active ingredient;
b. preparation of the hydrophilic phase by dissolving the chitosan and the polyoxyalkylenated compound in water;
c. heating both phases to a temperature between 40 and 60°C;
d. mixing the lipophilic and hydrophilic phase by stirring with spontaneous formation of the nanocapsules; and
e. elimination of the organic solvent.

## Patentansprüche

1. System, das in einem wässrigen Medium dispergierte Nanokapseln mit einer mittleren Größe von weniger als 1 µm umfasst, wobei die Nanokapseln eine Struktur aufweisen, die Folgendes umfasst:
a. eine lipophile Phase, umfassend:
a.1) wenigstens eine Phospholipidkomponente; und
a.2) ein Öl, das eine oder mehrere gesättigte und/oder ungesättigte C₁₂-C₂₄-kettige Fettsäuren oder Derivate davon umfasst;
b. eine hydrophile Phase, umfassend:
b.1) Chitosan oder ein Derivat davon; und
b.2) eine polyoxyalkylenierte Verbindung.

2. System gemäß Anspruch 1, wobei die Phospholipidkomponente Phosphoglyceride umfasst, die vorzugsweise aus Phosphatidylcholin, Phosphatidylethanolamin, Lysophosphatidylcholin, Phosphatidinsäure und Gemischen davon ausgewählt sind.

3. System gemäß Anspruch 2, wobei es sich bei der Phospholipidkomponente um Lecithin, vorzugsweise Sojalecithin, handelt.

4. System gemäß Anspruch 3, wobei das Lecithin einen Phosphatidylcholin-Anteil zwischen 35 und 75 Gew.-%, vorzugsweise zwischen 35 und 55 Gew.-%, aufweist.

5. System gemäß einem der Ansprüche 1 bis 4, wobei es sich bei dem Öl um Ricinusöl handelt.

6. System gemäß einem der Ansprüche 1 bis 5, wobei die polyoxyalkylenierte Verbindung ein Polyoxyethylen oder ein Ethylenoxid-Propylenoxid-Copolymer ist.

7. System gemäß Anspruch 6, wobei es sich bei der polyoxyalkylenierten Verbindung um ein Poloxamer und vorzugsweise Poloxamer 188 handelt.

8. System gemäß einem der Ansprüche 1 bis 7, wobei der Anteil der Phospholipidkomponente in dem System zwischen 0,1 und 10 Gew.-% liegt, bezogen auf das Gesamtgewicht einschließlich des Wassers, vorzugsweise zwischen 0,5 und 5 Gew.-%, besonders bevorzugt zwischen 1 und 2,5 Gew.-%.

9. System gemäß einem der Ansprüche 1 bis 8, wobei der Anteil von Öl in dem System zwischen 1 und 20 Gew.-% liegt, bezogen auf das Gesamtgewicht einschließlich des Wassers, vorzugsweise zwischen 2,5 und 10 Gew.-%, besonders bevorzugt zwischen 4,5 und 5,5 Gew.-%.

10. System gemäß einem der Ansprüche 1 bis 9, wobei der Anteil von Chitosan in dem System zwischen 0,05 und 10 Gew.-% liegt, bezogen auf das Gesamtgewicht einschließlich des Wassers, vorzugsweise zwischen 0,1 und 2 Gew.-%, besonders bevorzugt zwischen 0,25 und 1 Gew.-%.

11. System gemäß einem der Ansprüche 1 bis 10, wobei der Anteil der polyoxyalkylenierten Verbindung in dem System zwischen 0,05 und 10 Gew.-% liegt, bezogen auf das Gesamtgewicht einschließlich des Wassers, vorzugsweise zwischen 0,1 und 5 Gew.-%, besonders bevorzugt zwischen 1 und 2,5 Gew.-%.

12. System gemäß einem der Ansprüche 1 bis 11, wobei das wässrige Medium weiterhin ein verdickendes hydrophiles Polymer umfasst.

13. System gemäß Anspruch 12, wobei das verdickende Polymer aus Polyvinylalkohol, Polyvinylpyrrolidon, Chitosan, Poloxameren, Polyethylenglycol und Gemischen davon ausgewählt ist.

14. System gemäß einem der Ansprüche 1 bis 13, das weiterhin Hyaluronsäure oder eines ihrer Salze umfasst.

15. System gemäß einem der Ansprüche 1 bis 14, wobei die Nanokapseln eine mittlere Größe zwischen 1 und 999 nm, vorzugsweise zwischen 50 und 800 nm, besonders bevorzugt zwischen 200 und 700 nm aufweisen.

16. System gemäß einem der Ansprüche 1 bis 15, wobei die Nanokapseln eine positive Oberflächenladung haben, die vorzugsweise durch ein Zetapotential zwischen +1 mV und +70 mV dargestellt wird.

17. System gemäß einem der Ansprüche 1 bis 16, das weiterhin ein lipophiles Molekül in der lipophilen Phase der Nanokapseln umfasst, das aus der Gruppe ausgewählt ist, die aus Proteinen, Peptiden, Lipiden, lipophilisierten Oligonucleotiden, Corticosteroiden, lipophilen Vitaminen, wie A, D, K und E, fungiziden Mitteln, bakteriostatischen Mitteln, Heilmitteln, Antihistaminika, Anästhetika, juckreizstillenden Mitteln, antipsoriatischen Mitteln, Antibiotika, antiviralen Mitteln, antiseptischen Mitteln, Antiaknemitteln, depigmentierenden Mitteln, antiseborrhoischen Mitteln, immunsuppressiven Mitteln, nichtsteroidalen entzündungshemmenden Wirkstoffen (NSAIDs) und von *Capsicum* abgeleiteten entzündungshemmenden Mitteln besteht.

18. System gemäß Anspruch 17, wobei es sich bei dem lipophilen Molekül um Cyclosporin handelt.

19. System gemäß Anspruch 17, wobei es sich bei dem lipophilen Molekül um Hydrocortison, Prednison, Fluticason, Prednisolon, Triamcinolon, Triamcinolonacetonid, Dexamethason, Betamethason, Betamethasonvalerat, Beclomethason, Beclomethasondipropionat, vorzugsweise Betamethasonvalerat, handelt.

20. Pharmazeutische Zusammensetzung, die ein System, wie es in einem der Ansprüche 1 bis 19 definiert ist, und ein biologisch aktives Molekül, das Krankheiten verhindern, lindern oder heilen kann, umfasst.

21. Zusammensetzung gemäß Anspruch 20 zur Verabreichung auf oralem, bukkalem, sublingualem, topischem, okularem, nasalem oder vaginalem Weg.

22. Kosmetische Zusammensetzung, die ein System, wie es in einem der Ansprüche 1 bis 19 definiert ist, umfasst.

23. Zusammensetzung gemäß Anspruch 22, die ein kosmetisch annehmbares lipophiles Molekül umfasst, das aus Emollentien, Konservierungsmitteln, Duftstoffen, Antiaknemitteln, Fungiziden, Antioxidantien, Deodorantien, Antiperspirantien, Antischuppenmitteln, depigmentierenden Mitteln, antiseborrhoischen Mitteln, Farbstoffen, Bräunungslotionen, UV-Licht-Absorbern, Enzymen und Duftstoffen ausgewählt ist.

24. Verfahren zur Herstellung eines Systems gemäß einem der Ansprüche 1 bis 19, umfassend:
a. Herstellen der lipophilen Phase durch Auflösen der Phospholipidkomponente und des Öls in einem organischen Lösungsmittel und gegebenenfalls eines lipophilen Moleküls als Wirkstoff;
b. Herstellen der hydrophilen Phase durch Auflösen des Chitosans und der polyoxyalkylenierten Verbindung in Wasser;
c. Erhitzen beider Phasen auf eine Temperatur zwischen 40 und 60 °C;
d. Mischen der lipophilen und der hydrophilen Phase durch Rühren unter spontaner Bildung der Nanokapseln; und
e. Entfernen des organischen Lösungsmittels.

## Revendications

1. Système comprenant des nanocapsules ayant une taille moyenne inférieure à 1 µm dispersées dans un milieu aqueux, dans lequel les nanocapsules ont une structure comprenant :
a. une phase lipophile comprenant :
a.1) au moins, un composant phospholipide ; et
a.2) une huile comprenant un ou plusieurs acides gras saturés et/ou insaturés à chaîne en C₁₂ à C₂₄ ou des dérivés de ceux-ci ;
b. une phase hydrophile comprenant :
b.1) le chitosane ou un dérivé de celui-ci ; et
b.2) un composé polyoxyalkyléné.

2. Système selon la revendication 1, dans lequel le composant phospholipide comprend des phospho-glycérides, de préférence choisis parmi la phosphatidylcholine, la phosphatidyléthanolamine, la lysophosphatidylcholine, l'acide phosphati-dique et des mélanges de ceux-ci.

3. Système selon la revendication 2, dans lequel le composant phospholipide est la lécithine, de préférence la lécithine de soja.

4. Système selon la revendication 3, dans lequel la lécithine a une proportion de phosphatidyl-choline entre 35 % et 75 % en poids, de préférence entre 35 % et 55 %.

5. Système selon l'une quelconque des revendica-tions 1 à 4, dans lequel l'huile est de l'huile de ricin.

6. Système selon l'une quelconque des revendica-tions 1 à 5, dans lequel le composé polyoxy-alkyléné est un polyoxyéthylène ou est un copoly-mère oxyde d'éthylène - oxyde de propylène.

7. Système selon la revendication 6, dans lequel le composé polyoxyéthyléné est un poloxamère, de préférence le poloxamère 188.

8. Système selon l'une quelconque des revendica-tions 1 à 7, dans lequel la proportion en composant phospholipide dans le système se trouve entre 0,1 % et 10 % en poids par rapport au poids total, eau comprise, de préférence entre 0,5 % et 5 %, de manière davantage préférée entre 1 % et 2,5 % en poids.

9. Système selon l'une quelconque des revendica-tions 1 à 8, dans lequel la proportion en huile dans le système se trouve entre 1 % et 20 % en poids par rapport au poids total, eau comprise, de préférence entre 2,5 % et 10 %, de manière davantage préférée entre 4,5 % et 5,5 % en poids.

10. Système selon l'une quelconque des revendica-tions 1 à 9, dans lequel la proportion en chito-sane dans le système se trouve entre 0,05 % et 10 % en poids par rapport au poids total, eau comprise, de préférence entre 0,1 % et 2 %, de manière davantage préférée entre 0,25 % et 1 % en poids.

11. Système selon l'une quelconque des revendica-tions 1 à 10, dans lequel la proportion en composé polyoxyalkyléné dans le système se trouve entre 0,05 % et 10 % en poids par rapport au poids total, eau comprise, de préférence entre 0,1 % et 5 %, de manière davantage préférée entre 1 % et 2,5 %.

12. Système selon l'une quelconque des revendica-tions 1 à 11, dans lequel le milieu aqueux comprend en outre un polymère hydrophile apportant de la viscosité.

13. Système selon la revendication 12, dans lequel le polymère apportant de la viscosité est choisi parmi l'alcool polyvinylique, la polyvinyl-pyrrolidone, le chitosane, des poloxamères, du polyéthylène glycol et des mélanges de ceux-ci.

14. Système selon l'une quelconque des revendica-tions 1 à 13, comprenant en outre l'acide hyaluronique ou l'un quelconque de ses sels.

15. Système selon l'une quelconque des revendica-tions 1 à 14, dans lequel les nanocapsules ont une taille moyenne entre 1 et 999 nm, de préférence entre 50 et 800 nm, de manière davantage préférée entre 200 et 700 nm.

16. Système selon l'une quelconque des revendica-tions 1 à 15, dans lequel les nanocapsules ont une charge superficielle positive, de préférence représentée par un potentiel zêta entre + 1 mV et + 70 mV.

17. Système selon l'une quelconque des revendica-tions 1 à 16, comprenant en outre une molécule lipophile dans la phase lipophile des nano-capsules choisie dans le groupe constitué des protéines, des peptides, des lipides, des oligo-nucléotides lipophilisés, des corticostéroïdes, des vitamines lipophiles, telles que les vita-mines A, D, K et E, des agents antifongiques, des agents bactériostatiques, des agents curatifs, des agents antihistaminiques, des agents anesthé-siques, des agents antiprurigineux, des agents antipsoriasis, des agents antibiotiques, des agents antiviraux, des agents antiseptiques, des agents anti-acnéiques, des agents de dépigmentation, des agents antiséborrhéiques, des agents immunosuppresseurs, des médicaments anti-inflam-matoires non stéroïdiens (AINS), des anti-inflammatoires dérivés du Capsicum.

18. Système selon la revendication 17, dans lequel la molécule lipophile est une cyclosporine.

19. Système selon la revendication 17, dans lequel la molécule lipophile est l'hydrocortisone, la prednisone, la fluticasone, la prednisolone, la triamcinolone, un acétonide de la triamcino-lone, la dexaméthasone, la bétaméthasone, le valérate de bétaméthasone, la béclométhasone, le dipropionate de béclométhasone, de préférence le valérate de bétaméthasone.

20. Composition pharmaceutique comprenant un sys-tème tel que celui défini dans l'une quelconque des revendications 1 à 19 et une molécule biologiquement active capable de prévenir, soulager ou guérir des maladies.

21. Composition selon la revendication 20, destinée à être administrée par voie orale, buccale, sublinguale,topique,oculaire,nasale ou vaginale.

22. Composition cosmétique comprenant un système tel que celui défini dans l'une quelconque des revendications 1 à 19.

23. Composition selon la revendication 22, comprenant une molécule lipophile cosmétiquement acceptable choisie parmi des agents émollients, des conservateurs, des substances parfumantes, des agents anti-acnéiques, des agents anti-fongiques, des antioxydants, des déodorants, des anti-transpirants, des agents antipelliculaires, des agents de dépigmentation, des agents anti-séborrhéiques, des colorants, des laits solaires, des agents absorbants la lumière UV, des enzymes et des substances parfumantes.

24. Procédé de préparation d'un système selon l'une quelconque des revendications 1 à 19, comprenant les étapes consistant à :
a. préparer la phase lipophile en dissolvant le composant phospholipide et l'huile dans un solvant organique, et éventuellement une molé-cule lipophile comme principe actif ;
b. préparer la phase hydrophile en dissolvant le chitosane et le composé polyoxyalkyléné dans l'eau ;
c. chauffer les deux phases à une température entre 40 et 60°C ;
d. mélanger la phase lipophile et la phase hydro-phile en les agitant formant ainsi spontané-ment les nanocapsules ; et
e. éliminer le solvant organique.
